# EUROPEAN PATENT SPECIFICATION

(11) **EP 3 758 634 B1**
(45) Date of publication and mention of the grant of the patent: **31.12.2025**
(21) Application number: 19710881.4
(22) Date of filing: 26.02.2019
(51) Int. Cl.: A61B 17/70, A61F 2/30, A61F 2/44, B33Y 80/00, A61L 31/14, A61L 31/02

(54) **SPINAL IMPLANTS WITH CUSTOM DENSITY AND 3-D PRINTING OF SPINAL IMPLANTS**
WIRBELSÄULENIMPLANTATE MIT ANGEPASSTER DICHTE UND 3D-DRUCKEN VON WIRBELSÄULENIMPLANTATEN
IMPLANTS RACHIDIENS À DENSITÉ PERSONNALISÉE ET IMPRESSION 3D D'IMPLANTS RACHIDIENS

(30) Priority: 26.02.2018 US 201862635147 P; 08.05.2018 US 201862668499 P
(43) Date of publication of application: 06.01.2021
(73) Proprietor: VB SPINE US OPCO LLC, Wilmington, DE 19801 (US)
(72) Inventor: MORRISON, Thomas, Atlanta, GA 30328-5334 (US); WOODS, Richard, W., Catonsville, MD 21228 (US); PELLEGRINO, Richard, Leesburg, VA 20175 (US); PROSSER, Michael, Herndon, VA 20170 (US)
(74) Representative: Regimbeau
(86) International application number: PCT/US2019/019622
(87) International publication number: WO 2019/165445

(56) References cited:
- WO-A1-2017/004483
- WO-A1-2017/151548
- US-A1- 2016 213 487

## Description

### CROSS-REFERENCE TO RELATED APPLICATIONS

This application claims the benefit of the filing date of U.S. Provisional Patent Application No. 62/635,147 filed February 26, 2018 and U.S. Provisional Patent Application No. 62/668,499 filed May 8, 2018.

### FIELD OF THE INVENTION

The present invention relates to a method of manufacturing a spinal rod.

### BACKGROUND OF THE INVENTION

The spinal column is a complex system of bones and connective tissues that provide support for the human body and protection for the spinal cord and nerves. The adult spine is comprised of an upper and lower portion. The upper portion contains twenty-four discrete bones, which are subdivided into three areas including seven cervical vertebrae, twelve thoracic vertebrae and five lumbar vertebrae. The lower portion is comprised of the sacral and coccygeal bones. The cylindrical shaped bones, called vertebral bodies, progressively increase in size from the upper portion downwards to the lower portion.

An intervertebral disc along with two posterior facet joints cushion and dampen the various translational and rotational forces exerted upon the spinal column. The intervertebral disc is a spacer located between two vertebral bodies. The facets provide stability to the posterior portion of adjacent vertebrae. The spinal cord is housed in the canal of the vertebral bodies. It is protected posteriorly by the lamina. The lamina is a curved surface with three main protrusions. Two transverse processes extend laterally from the lamina, while the spinous process extends caudally and posteriorly. The vertebral bodies and lamina are connected by a bone bridge called the pedicle.

The spine is a flexible structure capable of a large range of motion. There are various disorders, diseases, and types of injury, which restrict the range of motion of the spine or interfere with important elements of the nervous system. The problems include, but are not limited to, scoliosis, kyphosis, excessive lordosis, spondylolisthesis, slipped or ruptured discs, degenerative disc disease, vertebral body fracture, and tumors. Persons suffering from any of the above conditions may experience extreme or debilitating pain and diminished nerve function. These conditions and their treatments can be further complicated if the patient is suffering from osteoporosis, or bone tissue thinning and loss of bone density.

Spinal discs between the endplates of adjacent vertebrae in a spinal column of the human body provide critical support. However, due to injury, degradation, disease or the like, these discs can rupture, degenerate, and/or protrude to such a degree that the intervertebral space between adjacent vertebrae collapses as the disc loses at least a part of its support function. This can cause impingement of the nerve roots and severe pain.

In some cases, surgical correction may be required. Some surgical corrections include the removal of the natural spinal disc from between the adjacent vertebrae. In order to preserve the intervertebral disc space for proper spinal column function, an interbody spacer can be inserted between the adjacent vertebrae.

Typically, a prosthetic implant is inserted between the adjacent vertebrae and may include pathways that permit bone growth between the adjacent vertebrae until they are fused together. However, there exists a possibility that conventional prosthetic implants may not provide a fusion due to various conditions and factors, including the fact that the implant does not allow optimal space for bone ingrowth and the implant does not mimic bone density sufficiently to allow for the creation of bone growth factors. In these cases, the body rejects the implant and a non-union (no fusion) occurs. When there is a non-union, the implants may be dislodged or moved from their desired implanted location due to movement by the patient or insufficient bone ingrowth.

Also, WO2017/004483 discloses a pedicle screw rod having a elongate rod and a sensor configured to measure fusion of one or more vertebral bodies as a function of strain of the elongate rod.

Therefore, a need exists for a spinal implant that can mimic the density of bone and allow for optimal bone ingrowth and provide a solid fusion of the vertebral segments. In addition, it is desired that an implant be utilized to prevent expulsion of the interbody device by utilizing a spinal plate.

### BRIEF SUMMARY OF THE INVENTION

The present invention is defined in claim 1 while preferred embodiments are set forth in the dependent claims.

The term "embodiment" used in the present specification does not necessarily indicate ways of carrying out the invention claimed but also examples which aid understanding the invention.

According to the present invention, a method of manufacturing a spinal rod comprises the following steps: using a software for identifying a required geometric shape of the spinal rod by obtaining a digital image of a patient and overlaying a representative spinal rod on a plurality of anatomical landmarks on the digital image; and forming at least part of the spinal rod using a directed energy deposition process, a powder bed fusion process, a binder jetting process or a shape deposition manufacturing process, the process comprising: selecting a molybdenum rhenium alloy consisting of 52% to 70% molybdenum and 30% to 48% rhenium, the molybdenum rhenium alloy being used to form the at least part of the spinal rod; and curing a plurality of layers of the selected molybdenum rhenium alloy (implicitly disclosed since D1 describes additive manufacturing e.g. 3D-printing and sintering technologies like light, laser, electrical sintering; see para. 39) to form the at least part of the spinal rod having the required geometric shape without the need for bending or other post machining processes to conform to a patient's body.

According to a non-claimed embodiment of the present disclosure, a spinal implant includes a body portion defining a longitudinal axis, the body portion including a distal end portion, a proximal end portion, opposed side surfaces that extend between the distal and proximal end portions, and top and bottom surfaces configured and adapted to engage vertebral bodies. The top and bottom surfaces have a surface roughness between about 3-4 µm. The spinal implant includes a cavity extending through the top and bottom surfaces defining a surface area that is at least 25% of a surface area of the top surface or the bottom surface. The spinal implant includes first orifices defined through the top surface and second orifices defined through the bottom surface. Each second orifice is connected to a first orifice by a channel.

In embodiments, one of the first orifices may be offset from one of the second orifices.

In embodiments, the spinal implant may have a first plurality of enlarged orifices is defined through one of the top or bottom surfaces and may have a second plurality of enlarged orifices is defined through the other of the top or bottom surfaces. An enlarged orifice of the second plurality of enlarged orifices may include a diameter that is different than a diameter of an enlarged orifice of the first plurality of enlarged orifices. The enlarged orifice of the first plurality of enlarged orifices or the enlarged orifice of the second plurality of enlarged orifices may include a circular cross-section.

In embodiments, the enlarged orifice of the first plurality of enlarged orifices may include a diamond-shaped cross-section and the enlarged orifice of the second plurality of enlarged orifices may include a diamond-shaped cross-section. Each enlarged orifice of the first and second pluralities of enlarged orifices may include a diamond-shaped cross-section.

In embodiments, the spinal implant may have third orifices that are defined through at least one of the opposed side surfaces. One of the third orifices may include a cross-section different than one of the first orifices or one of the second orifices. Opposed openings of one of the third orifices may be offset with respect to each other. One of the third orifices may include a diamond-shaped cross-section.

In embodiments, the spinal implant may have a third plurality of enlarged orifices defined through one of the opposed side surfaces. One enlarged orifice of the third plurality of enlarged orifices may include a diamond-shaped cross-section.

In embodiments, the spinal implant may be formed using an additive manufacturing process.

In embodiments, the spinal implant may have a through-bore defined through the spinal implant. An interior dimension of the through-bore may increase in a direction towards each respective opposed side surface. A bevel may be interposed between each opposed side surface and an interior wall defining the through-bore.

In embodiments, the spinal implant is formed from titanium.

In embodiments, one of the first orifices has a cross-sectional configuration different from that of one of the second orifices.

According to another embodiment of the present disclosure, a spinal implant includes a body portion that defines a longitudinal axis. The body portion includes a distal end portion, a proximal end portion, opposed side surfaces that extend between the distal and proximal end portions, and top and bottom surfaces configured and adapted to engage vertebral bodies. The top and bottom surfaces have a surface roughness between about 0.1-50 µm. The implant also includes first, second, third and fourth orifices. The first orifices are defined through the top surface and have a first shape. The second orifices are defined through the bottom surface and have the first shape. Each second orifice is connected to a respective first orifice by one channel of a first plurality of channels. The third orifices are defined through a first side surface of the opposed side surfaces and have a second shape. The fourth orifices are defined through a second side surface of the opposed side surfaces and have the second shape. Each fourth orifice is connected to a respective third orifice by one channel of a second plurality of channels. Additionally, the first shape is different from the second shape and at least one of the second plurality of channels is offset from each of the first plurality of channels.

In some embodiments, one of the first orifices may be offset from one of the second orifices. In some embodiments, the one of the first orifices may be in communication with the one of the second orifices through a first channel of the first plurality of channels. In some embodiments, at least one channel of the first plurality of channels may be oriented at an acute angle relative to the top surface. In some embodiments, the first orifices may have a first density and at least one of the second, third and fourth orifices may have a second density, the first density different from the second density. In some embodiments, at least one of the first shape and the second shape may include a circular cross-section. In some embodiments, at least one of the first shape and the second shape may include a diamond-shaped cross-section. In some embodiments, one of the first shape and the second shape may include a circular cross-section and the other of the first shape and the second shape may include a diamond-shaped cross-section. In some embodiments, the top surface or the bottom surface may include fifth orifices having a third shape different from the first shape. In some embodiments, the first orifices may have a first density and the fifth orifices may have a second density different from the first density. In some embodiments, at least one of the first, second, third or fourth orifices may have a diameter between about 300-700 µm.

In one non-claimed example, a method of manufacturing a spinal rod includes: identifying a geometric shape of the spinal rod and forming at least part of the spinal rod using an additive manufacturing process. The additive manufacturing process includes: selecting a material from which the at least part of spinal rod will be formed and curing a plurality of layers of the selected material to form the spinal rod according to the identified geometric shape.

In some examples, selecting the material may include selecting a molybdenum rhenium alloy from which the at least part of the spinal rod will be formed. In some examples, selecting the material may include selecting a molybdenum rhenium alloy containing between 40 and 51% molybdenum and rhenium. In some examples, selecting the material may include selecting titanium or a titanium alloy from which the at least part of the spinal rod will be formed. In some examples, the method may include forming a second part of the rod using a process other than additive manufacturing. In some examples, the method may include forming a second part of the rod separate from the at least part of the spinal rod, the second part formed through the selection of a second material different than the material. In other examples, the method of manufacture may be performed for implants other than spinal rods.

### BRIEF DESCRIPTION OF THE DRAWINGS

Various examples of the present disclosure are described hereinbelow with reference to the drawings, which are incorporated in and constitute a part of this specification, wherein
FIG. 1 is a perspective view of an embodiment of a spinal implant provided in accordance with the present disclosure;
FIG. 2 is a top view of the spinal implant of FIG. 1;
FIG. 3 is a rear view of the spinal implant of FIG. 1;
FIG. 4 is a side view of the spinal implant of FIG. 1;
FIG. 5A is a cross-sectional view taken along the section line 5-5 of FIG. 3;
FIG. 5B is a cross-sectional view of a different embodiment of a spinal implant similar to the spinal implant of FIG. 3 taken along the section line 5-5 of FIG. 3;
FIG. 6 is a perspective view of another embodiment of a spinal implant provided in accordance with the present disclosure;
FIG. 7 is a top view of the spinal implant of FIG. 6;
FIG. 8 is a rear view of the spinal implant of FIG. 6;
FIG. 9 is a side view of the spinal implant of FIG. 6;
FIG 10 is a front view of the spinal implant of FIG. 6;
FIG. 11 is a perspective view of another embodiment of a spinal implant provided in accordance with the present disclosure;
FIG. 12 is a top view of the spinal implant of FIG. 11;
FIG. 13 is a rear view of the spinal implant of FIG. 11;
FIG. 14 is a side view of the spinal implant of FIG. 11;
FIG. 15A is a cross-sectional view taken along the section line 15-15 of FIG. 13;
FIG. 15B is a cross-sectional view of a different embodiment of a spinal implant similar to the spinal implant of FIG. 13 taken along the section line 15-15 of FIG. 13;
FIG. 16 is a perspective view of another embodiment of a spinal implant provided in accordance with the present disclosure;
FIG. 17 is a top view of the spinal implant of FIG. 16;
FIG. 18 is a rear view of the spinal implant of FIG. 16;
FIG. 19 is a side view of the spinal implant of FIG. 16;
FIG. 20A is a cross-sectional view taken along the section line 20-20 of FIG. 18;
FIG. 20B is a cross-sectional view of a different embodiment of a spinal implant similar to the spinal implant of FIG. 18 taken along the section line 20-20 of FIG. 18;
FIG. 21 is a perspective view of yet another embodiment of a spinal implant provided in accordance with the present disclosure;
FIG. 22 is a side view of the spinal implant of FIG. 21;
FIG. 23 is a top view of the spinal implant of FIG. 21;
FIG. 24 is a top view of a different embodiment of a spinal implant similar to the spinal implant of FIG. 21;
FIG. 25 is a side view of a different embodiment of a spinal implant similar to the spinal implant of FIG. 21;
FIG. 26 is a front, cross-sectional view, of the spinal implant of FIG. 21 taken along section line 26-26 of FIG. 22;
FIG. 27 is a bottom, cross-sectional view, of the spinal implant of FIG. 21, taken along section line 27-27 of FIG. 23;
FIG. 28 is a perspective view of a spinal rod provided in accordance with the present disclosure;
FIG. 29 is a perspective view of another spinal rod provided in accordance with the present disclosure, shown with bends formed along the length thereof;
FIG. 30 is a perspective view of a bone screw assembly provided in accordance with the present disclosure, shown with parts separated;
FIG. 30A is a cross-sectional view of the bone screw assembly of FIG. 30, taken along section line 30A-30A of FIG. 30; and
FIG. 31 is a perspective view of the bone screw assembly of FIG. 30, shown as being formed from an additive manufacturing process.

### DETAILED DESCRIPTION

Embodiments of the present disclosure are now described in detail with reference to the drawings in which like reference numerals designate identical or corresponding elements in each of the several views. As commonly known, the term "clinician" refers to a doctor, a nurse, or any other care provider and may include support personnel. Additionally, the term "proximal" refers to the portion of the device or component thereof that is closer to the clinician and the term "distal" refers to the portion of the device or component thereof that is farther from the clinician. In addition, the term "cephalad" is known to indicate a direction toward a patient's head, whereas the term "caudal" indicates a direction toward the patient's feet. Further still, the term "lateral" is understood to indicate a direction toward a side of the body of the patient, i.e., away from the middle of the body of the patient. The term "posterior" indicates a direction toward the patient's back, and the term "anterior" indicates a direction toward the patient's front. Additionally, terms such as front, rear, upper, lower, top, bottom, and similar directional terms are used simply for convenience of description and are not intended to limit the disclosure. In the following description, well-known functions or constructions are not described in detail to avoid obscuring the present disclosure in unnecessary detail.

Reference may be made to U.S. Patent Application Publication No. 2016/0213487, titled "Spinal Implant," filed on January 27, 2016, U.S. Patent Application Publication No. 2016/0213488, titled "Interbody Spacer," filed on January 27, 2016, U.S. Patent Application Publication No. 2016/0213486, titled "Interbody Spacer," filed on January 27, 2016, U.S. Patent Application Publication No. 2016/0213405, titled "Vertebral Plate Systems and Methods of Use," filed on January 27, 2016, and U.S. Patent Application Publication No. 2016/0213485, titled "Interbody Spacer," filed on January 27, 2016, for exemplary spinal implants and methods of construction from which the spinal implants and spinal rods disclosed herein may be formed.

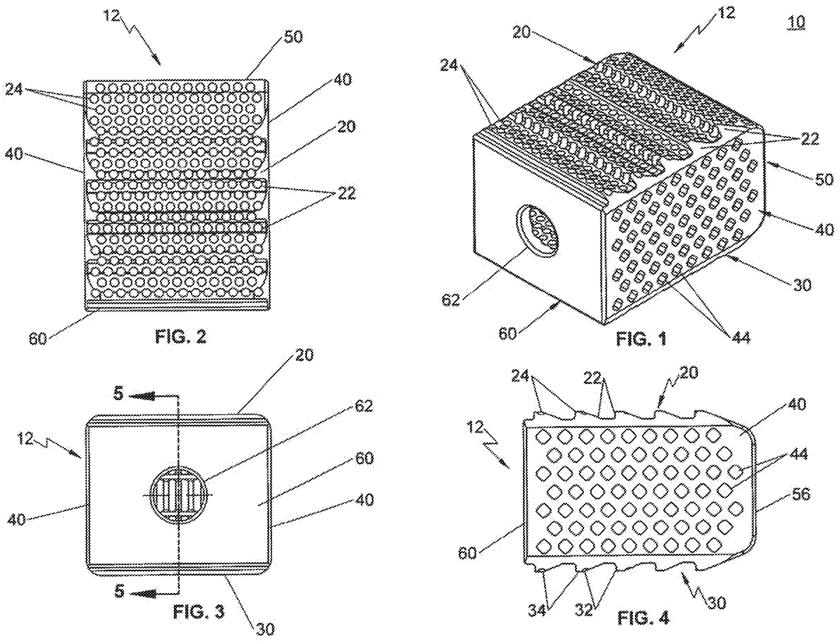

Referring now to FIGS. 1-4, a spinal implant 10 is provided in accordance with the present disclosure and includes a body 12 having a top surface 20, a bottom surface 30, side surfaces 40, a front surface 50, and a rear surface 60. The edges between each of the surfaces of the body 12 may include a bevel or a radius that provide a smooth transition between the adjacent surfaces of the body 12. The top and bottom surfaces 20, 30 are substantially parallel to one another and each includes engagement features 22, 32, respectively, that are configured to permit the spinal implant 10 to move in one direction, e.g., in a direction towards the front surface 20, and prevent or resist movement of the spinal implant 10 in the opposite direction, e.g., in a direction towards the rear surface 60. It is contemplated that the top and bottom surfaces 20, 30 may be disposed at an angle or curved relative to one another, e.g., in a lordotic or a kyphotic relationship to each other, such that the spinal implant 10 is substantially wedge shaped. As shown, the engagement features 22, 32 are rear facing teeth that are configured to engage endplates of adjacent vertebral bodies. The rear surface 60 defines a substantially circular engagement opening 62 that is engagable by a surgical instrument (not shown) to insert and/or reposition the surgical implant 10 between adjacent vertebral bodies.

The top surface 20, the bottom surface 30, and side surfaces 40 have a surface roughness that can promote bone growth and fusion with the spinal implant 10. The surface roughness may be in a range of about 0.10-50 µm, e.g., in a range of about 3-4 µm. In addition, the top surface 20, bottom surface 30, and side surfaces 40 define orifices 24, 34, and 44, respectively, which are sized to promote bone growth into the spinal implant 10. The orifices 24, 34, and 44 are typically circular to mimic bone growth along Haversian canals and lamellar structures of bone. The orifices 24, 34, and 44 may pass entirely through the body 12 of the spinal implant 10 extending orthogonal to the respective surface of the spinal implant 10. Each of the orifices 24 that pass through the top surface 20 may be aligned with a respective one of the orifices 34 that pass through the bottom surface 30. Each of the orifices 24 and 34 are offset from each of the orifices 44. The orifices 24, 34, and 44, have a diameter in the range of about 50-1000 µm, e.g., about 300-700 µm. The orifices 24, 34, and 44 may have varying sizes and shapes between the different surfaces 20, 30, 40 of the spinal implant 10. It is contemplated that the orifices 24, 34, and 44 may vary in size and shape on the same surface 20, 30, 40 of the spinal implant 10. For example, the orifices 24 and 34 are substantially circular in cross-section and the orifices 44 are substantially square in cross-section. The orifices 24, 34, 44 may reduce the density and stiffness of the spinal implant 10 and allow space for applying bone putty or the like to the spinal implant 10 to promote bone growth and fusion of the adjacent vertebral bodies to the spinal implant 10.

In addition, the spinal implant 10 may define connecting features (not explicitly shown) that further reduce the stiffness of the spinal implant 10. Further, the connecting features may reduce the scatter of the spinal implant 10 during a MRI or CT scan (e.g., when the spinal implant 10 is constructed from titanium). The connecting features also increase the interconnectedness of bone growth through and around the spinal implant 10 which may improve fusion to keep the spinal implant 10 in place and may reduce the chance of breakage of the spinal implant 10. The connecting features may be defined with a width or diameter in a range of about 150-450 µm, e.g., in a range of about 150-380 µm.

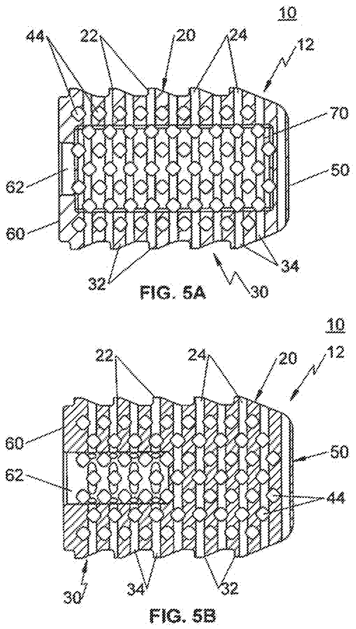

With additional reference to FIG. 5A, the body 12 is hollow and defines an internal cavity 70. As shown in FIG. 5A, each of the top surface 20, the bottom surface 30, side surfaces 40 (FIG. 3), the front surface 50, and the rear surface 60 are thin-walled to define the cavity 70 therebetween. Each of the top surface 20, the bottom surface 30, side surfaces 40 (FIG. 3), the front surface 50, and the rear surface 60 may have a thickness in a range of about 0.009 inches to about 0.020 inches. Alternatively, as shown in FIG. 5B, the body 12 may be substantially solid such that the engagement opening 62 extends into the body 12 towards the front surface 50. In such an embodiment, the engagement opening 62 is a blind hole and may extend in a range of about one quarter to one half of the length of the body 12.

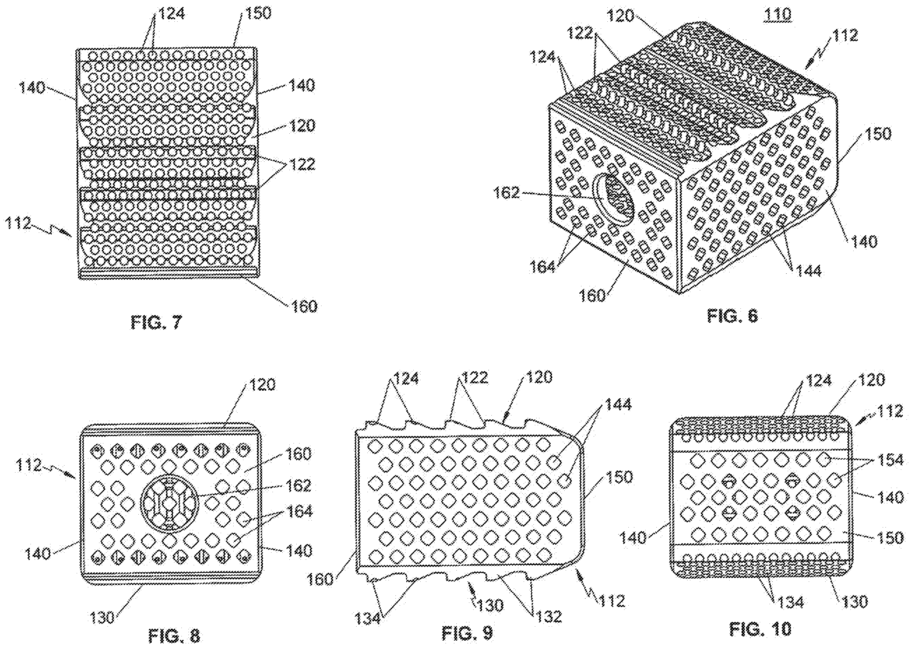

Referring now to FIGS. 6-10, another spinal implant 110 is provided in accordance with the present disclosure. The spinal implant 110 is similar to the spinal implant 10 detailed above with similar structures represented with reference numerals including a "1" preceding the previous reference numeral. Similar features will not be discussed in detail for reasons of brevity. The spinal implant 110 includes a body 112 having a top surface 120, a bottom surface 130, side surfaces 140, a front surface 150, and a rear surface 160. The top surface 120, bottom surface 130, side surfaces 140, the front surface 150, and the rear surface 160 define orifices 124, 134, 144, 154, and 164, respectively, which are sized to promote bone growth into the spinal implant 110. Each of the orifices 154 that pass through the front surface 150 are aligned with a respective one of the orifices 164 that pass through the rear surface 160. In addition, each of the orifices 154, 164 are offset from each of the orifices 124, 134 and each of the orifices 144.

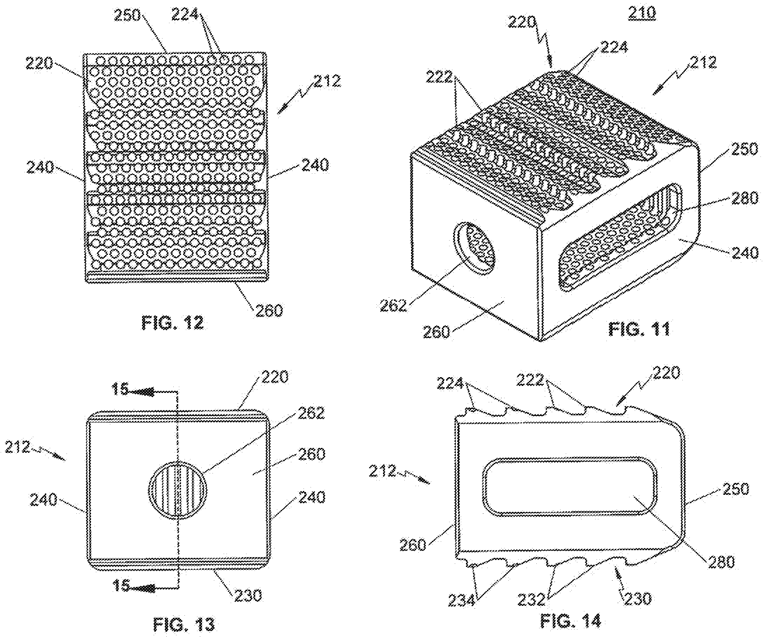

Referring now to FIGS. 11-14, another spinal implant 210 is provided in accordance with the present disclosure. The spinal implant 210 is similar to the spinal implant 10 detailed above with similar structures represented with reference numerals including a "2" preceding the previous reference numeral. Similar features will not be discussed in detail for reasons of brevity.

The spinal implant 210 includes a body 212 having a top surface 220, a bottom surface 230, side surfaces 240, a front surface 250, and a rear surface 260. The top surface 220 and the bottom surface 230 define orifices 224 and 234, respectively. The body 212 defines a lateral window 280 that passes through the side surfaces 240. The lateral window 280 is sized to promote bone growth and fusion with the spinal implant 210. The lateral window 280 may also reduce the density and stiffness of the body 212 of the spinal implant 210. The lateral window 280 may be vertically aligned with the engagement opening 262 of the rear surface 260.

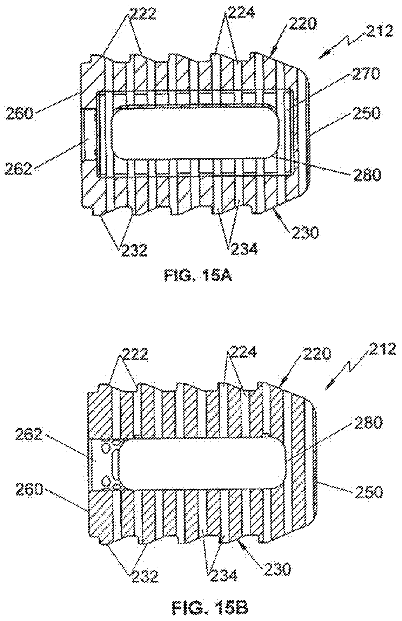

With additional reference to FIG. 15A, the body 212 is hollow and defines an internal cavity 270. As shown in FIG. 15A, each of the top surface 220, the bottom surface 230, side surfaces 240 (FIG. 11), the front surface 250, and the rear surface 260 are thin-walled to define the cavity 270 therebetween. Alternatively, as shown in FIG. 15B, the body 212 may be substantially solid such that the engagement opening 262 extends into the body 212 towards the front surface 250. In such an embodiment, the diameter of the engagement opening 262 may be substantially equal to a height of the lateral window 280.

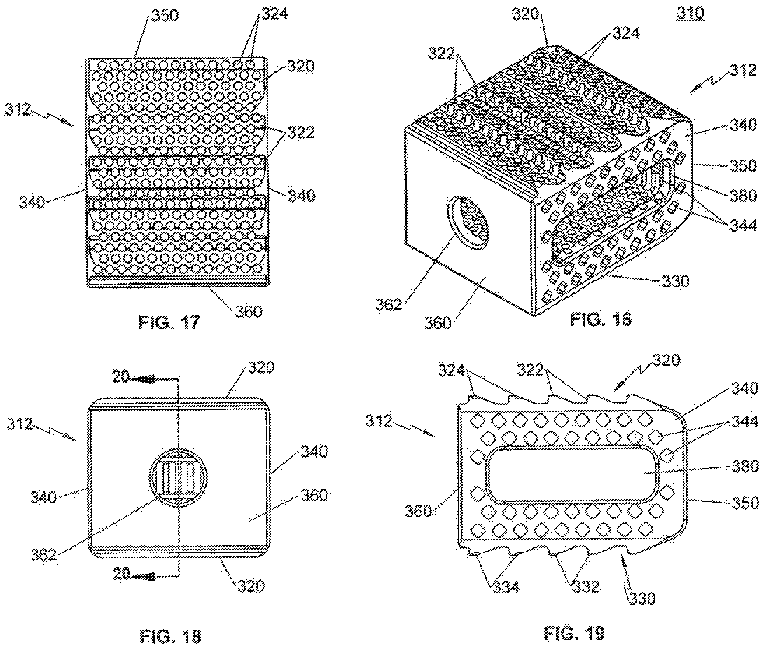

Referring now to FIGS. 16-19, another spinal implant 310 is provided in accordance with the present disclosure. The spinal implant 310 is similar to the spinal implant 10 detailed above with similar structures represented with reference numerals including a "3" preceding the previous reference numeral. Similar features will not be discussed in detail for reasons of brevity.

The spinal implant 310 includes a body 312 having a top surface 320, a bottom surface 330, side surfaces 340, a front surface 350, and a rear surface 360. The top surface 320, side surfaces 340, and the bottom surface 330 define orifices 324, 334, and 344, respectively. The spinal implant 310 defines a lateral window 380 that passes through the side surfaces 340 which is similar to the lateral window 280 of the body 212 of the spinal implant 210 detailed above.

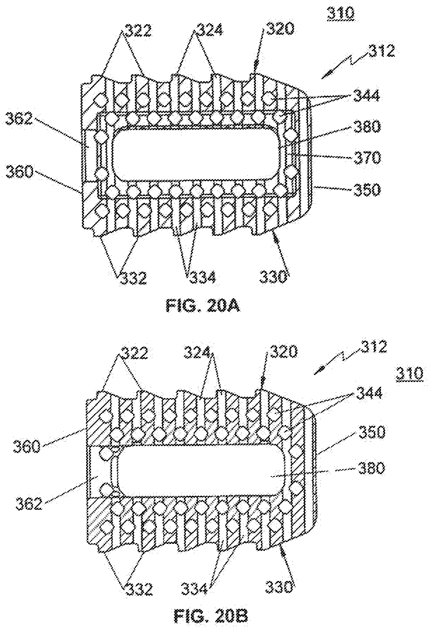

With additional reference to FIG. 20A, the body 312 is hollow and defines an internal cavity 370. As shown in FIG. 20A, each of the top surface 320, the bottom surface 330, side surfaces 340 (FIG. 16), the front surface 350, and the rear surface 360 are thin-walled to define the cavity 370 therebetween. Alternatively, as shown in FIG. 20B, the body 312 may be substantially solid such that the engagement opening 362 extends into the body 312 towards the front surface 350. In such an embodiment, the diameter of the engagement opening 362 may be substantially equal to a height of the lateral window 380.

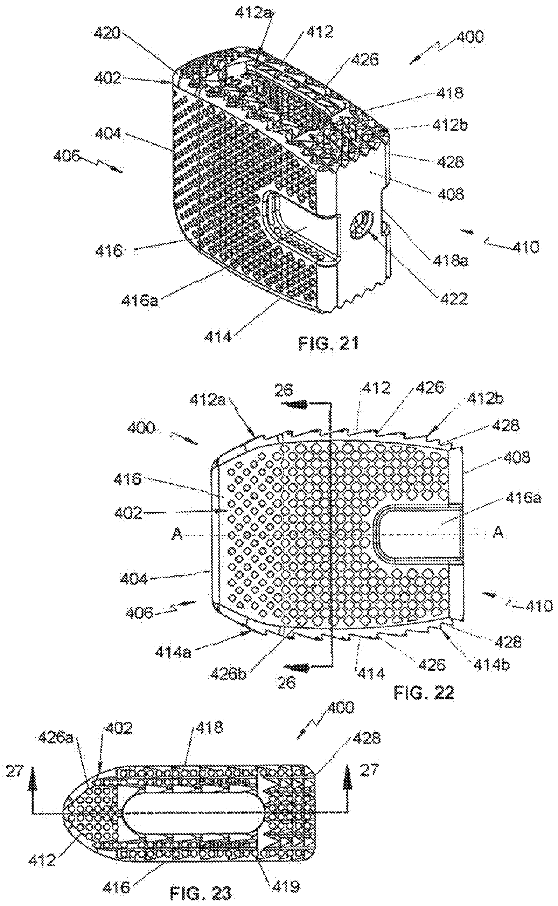

Referring to FIGS. 21-23, yet another embodiment of a spinal implant provided in accordance with the present disclosure is illustrated and generally identified by reference numeral 400. Spinal implant 400 includes a body 402 having a substantially contoured first end surface 404 at a distal or leading end 406 and a second end surface 408 opposite thereto at a proximal or trailing end 410, having a substantially planar configuration. Axis A-A is defined through a midpoint of first and second end surfaces 404, 408, respectively. Body portion 402 extends between first and second end surfaces 404, 408 to define respective top and bottom surfaces 412 and 414 (FIG. 22), respectively, as well as opposed side surfaces 416, 418 (FIG. 23). As best illustrated in FIG. 22, top and bottom surfaces 412, 414 include a generally convex or arcuate profile, each extending in a cephalad and caudal direction, respectively. Although shown and discussed as the top surface 412 being oriented in a cephalad direction and the bottom surface 414 being oriented in a caudal direction, the implant 400 may be positioned such that the top surface 412 in a caudal orientation and the bottom surface 414 is in a cephalad orientation. As can be appreciated, top and bottom surfaces 412, 414 may include a concave profile, a planar profile, or any combination thereof. In embodiments, top surface 412 may include a different profile than that of bottom surface 414. Additionally, it is contemplated that top and bottom surfaces 412, 414 may approximate towards each other in a distal direction along axis A-A (or vice versa), or may approximate towards each other in a direction from side surface 416 towards side surface 418 (or vice versa), or any combination thereof.

As best illustrated in FIG. 23, opposed side surfaces 416, 418 are substantially planar, although other configurations are also contemplated such as convex, concave, or the like. Opposed side surfaces 416, 418 approximate towards each other at distal end 406 along longitudinal axis A-A in order to facilitate insertion within the intervertebral space and enhance the atraumatic character of body portion 402. In this manner, the intersection of top and bottom surfaces 412, 414 with each of first and second end surfaces 404, 408 and opposed side surfaces 416, 418 may include a fillet or rounded configuration 420 to inhibit sharp edges from causing trauma to the surrounding tissue and/or vertebral bodies.

Referring again to FIG. 21, second end surface 408 includes an aperture 422 defined therethrough and extending along longitudinal axis A-A. Aperture 422 is configured for selective engagement with a suitable insertion tool (not shown), such as that described in U.S. Patent Application Serial No. 2012/0158062, filed October 11, 2011. In embodiments, aperture 422 may be threaded or otherwise include various features capable of selectively retaining a suitable insertion tool therein, such as a keyhole configuration, quarter turn configuration, or the like.

Each of opposed side surfaces 416, 418 include a corresponding depression or recess 416a, 418a defined therein adjacent second end surface 408. Recesses 416a, 418a extend along longitudinal axis A-A and are symmetrically disposed on each of opposed side surfaces 416, 418 to define a substantially I-shaped configuration to second end surface 408 at proximal end 410. In cooperation with aperture 422, the recesses 416a, 418a are further configured to enable engagement with stabilizing jaws of a suitable insertion instrument to facilitate the insertion of spinal implant 400.

Body 402 includes a through-bore or cavity 424 defined through top and bottom surfaces 412, 414, respectively. Although shown as having a generally oval configuration, it is contemplated that through-bore 424 may include any suitable shape, such as square, rectangular, circular, or the like, or may include a configuration similar to that of the outer perimeter of body 402. It is contemplated that through-bore 424 may receive allograft material, autograft material, calcium phosphate/bone marrow aspirate (BMA), autogenous material, synthetic materials comprised of a biocompatible, osteoconductive, osteoinductive, or osteogeneic material such as VITOSS^{®} Synthetic Cancellous Bone Void Filler material, or any other suitable biological material known in the art. Through-bore 424 includes a cross-sectional area or surface area that is greater than any orifice of the plurality of orifices or enlarged orifices detailed hereinbelow. In embodiments, through-bore 424 includes a surface area that is equal to or greater than 25% of the surface area of top surface 412 or bottom surface 414.

Top and bottom surfaces 412, 414 of body portion 402 are configured to engage respective endplates of adjacent vertebral bodies. In this manner, each of top and bottom surfaces 412, 414 include at least first and second surface regions 412a, 412b and 414a, 414b, respectively, which have distinct surface characteristics. As best illustrated in FIG. 22, first surface regions 412a, 414a are disposed distal to second surface regions 412b, 414b and include a surface characteristic that is different than that of second surfaces 412b, 414b. In embodiments, first surface regions 412a, 414a may include a same or similar surface characteristic to that of second surface regions 412b, 414b, or each of first and second surface regions 412a, 414a and 412b, 414b may include the same or different surface characteristics, or any combination thereof.

First surface regions 412a, 414a have a plurality of protrusions (i.e., teeth) or ridges 426 disposed thereon to aid in securing spinal implant 400 to each respective adjacent vertebral body and stability against fore and aft, oblique or side to side movement of spinal implant 400 within the intervertebral space. Specifically, ridges 426 frictionally engage endplates of adjacent vertebral bodies and inhibit movement of the spinal implant 400 with respect to the adjacent vertebral bodies. In embodiments, a longitudinal groove 419 (FIG. 23) may be defined between adjacent rows of protrusions 426, each of which extends along axis A-A. Each of second surface regions 412b, 414b includes substantially pyramidal protrusions 428, where each pyramidal protrusion 428 includes a plurality of protrusions or ridges disposed thereon to similarly aid in securing spinal implant 400 to each respective adjacent vertebral body. In particular, each pyramidal protrusion 428 includes opposed first and second faces that face, respectively, distally and proximally. Further, each pyramidal protrusion 428 has third and fourth faces that face, respectively, medially and laterally. For a detailed description of spinal implant having exemplary surface characteristics, reference can be made to U.S. Patent No. 8,801,791 to Soo et al.

Spinal implant 400 is constructed of a biocompatible material, such as commercially pure titanium or titanium alloy and includes a porosity capable of promoting bone ingrowth and fusion with spinal implant 400. In this manner, top and bottom surfaces 412, 414 and opposed side surfaces 416, 418 have a surface roughness that can promote bone growth and fusion with spinal implant 400. The surface roughness may be in a range of about 0.10-50 µm, and preferably in a range of about 3-4 µm. As can be appreciated, top and bottom surfaces 412, 414 and opposed side surfaces 416, 418 may include the same or different surface roughness's (i.e., the surface roughness of top surface 416 may be different than the surface roughness of bottom surface 414), or top and bottom surfaces 412, 414 and opposed side surfaces 416, 418 may not include a surface roughness; rather, top and bottom surfaces 412, 414 and opposed side surfaces 416, 418 may be smooth. In embodiments top and bottom surfaces 412, 414 and opposed side surfaces 416, 418 may include any combination of surface roughness or smooth surface.

Additionally, body 402 includes a plurality of orifices 426a and 426b defined through top and bottom surfaces 412, 414 and opposed side surfaces 416, 418, respectively, configured to promote bone ingrowth. Orifices 426a, 426b include a generally circular and diamond shaped cross-section, respectively, although other suitable cross-sections capable of promoting bone ingrowth are contemplated, such as oval, square, hexagonal, rectangular, or the like. The circular and diamond shaped-cross sections of orifices 426a, 426b, respectively, mimic bone growth along Haversian canals and lamellar structures of bone. In this manner, orifices 426a, 426b may pass entirely through top surface and bottom surfaces 412, 414 and opposed surfaces 416, 418, respectively. Alternatively, orifices 426a may be offset in relation to one another, and similarly with orifices 426b. In the interest of brevity, only orifices 426a will be described in detail herein below with respect to the offset nature of orifices 426a and 426b. An orifice 426a defined through bottom surface 414 will be offset from a corresponding orifice 426a defined through top surface 412. In embodiments, orifices 426a may be defined through top and bottom surfaces 412, 414 normal thereto or at angles relative thereto. In one non-limiting embodiment, orifices 426a are defined through top and bottom surfaces 412, 414 at angles incident relative to each other, thereby forming a chevron configuration. As can be appreciated, each of the orifices 426a and 426b formed through top and bottom surfaces 412, 414 and opposed side surfaces 416,418, respectively, form a respective channel therebetween, thereby interconnecting an orifice formed through top surface 416 and an orifice formed through bottom surface 414, or an orifice formed through side surface 416 and an orifice formed through side surface 418. It is contemplated that the density of orifices 426a may be different on top surface 412 than on bottom surface 414, or may increase or decrease in density at various locations on each of top and bottom surfaces 412, 414. Orifices 426a include a diameter in a range of about 50-1000 µm, although a diameter between 300-700 µm is preferable. As can be appreciated, for shapes other than circular, orifices 426a include a cross-sectional area in a range of about 0.0019 µm²-0.785 µm², although a cross-sectional area between 0.0707 µm²-0.385 µm² is preferable. As can be appreciated, the plurality of orifices 426a may include orifices 426a having varying sizes and shapes relative to each other. In embodiments, the orifices 426a defined through top surface 412 may include a different cross-section than those orifices 426a defined through bottom surface 414 (i.e., circular on top surface 412 while square on bottom surface 414, or vice versa). The plurality of orifices 426a reduce the density and stiffness of spinal implant 400 to enable the application of bone putty or the like (e.g., Bone Morphogenetic Proteins (BMP), etc.) to spinal implant 400 to promote bone ingrowth within spinal implant 400 and fusion to adjacent vertebral bodies. Bone ingrowth and fusion strengthens spinal implant 400. In this manner, the likelihood that micromotion would occur would likewise be reduced. In some embodiments, any number of the features of the orifices described above for implant 400 may be included in implants 10, 110, 210, 310, 500, 600.

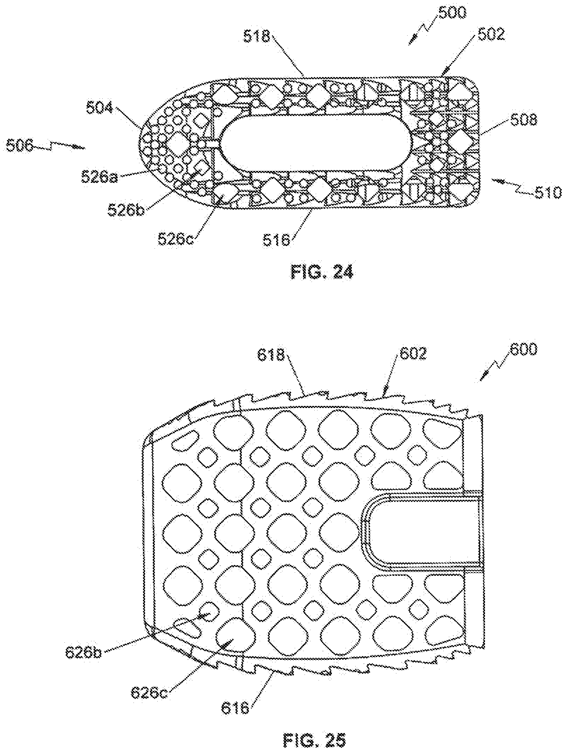

Referring to FIG. 24, another embodiment of a spinal implant provided in accordance with the present disclosure is illustrated and generally identified by reference numeral 500. Spinal implant 500 is substantially similar to spinal implant 400, and therefore, only the differences therebetween will be described in detail in the interest of brevity. Body 502 includes a first plurality of enlarged orifices 526c defined through top and bottom surfaces 512, 514. The first plurality of enlarged orifices 526c is arranged around the perimeter of body 502. In one non-limiting embodiment, the first plurality of enlarged orifices 526c are disposed approximately equidistant between opposed side surfaces 516, 518, through-bore 524, and first and second end surfaces 504, 508. A second plurality of enlarged orifices 526d is defined through top and bottom surfaces 512, 514 on each of the leading and trailing ends 508, 510, and includes a smaller diameter than that of the first plurality of enlarged orifices 526c. In this manner, the second plurality of enlarged orifices 526d is interposed between the first plurality of enlarged orifices 526c disposed on the leading and trailing ends 508, 510 and through-bore 524. Although illustrated as having a generally diamond shaped cross-section, it is contemplated that the first and second plurality of enlarged orifices 526c, 526d may include any suitable cross-section, such as circular, oval, square, hexagonal, rectangular, or the like. As can be appreciated, the first and second plurality of enlarged orifices 526c, 526d may be defined through top and bottom surfaces 512, 514 in any manner similar as described above with respect to spinal implant 400.

A plurality of orifices 526a is defined through top and bottom surfaces 512, 514, similarly to that described above with respect to spinal implant 400; however, the plurality of orifices 526a is interposed between each of the first and second plurality of enlarged orifices 526c, 526d.

Turning now to FIG. 25, still another embodiment of a spinal implant provided in accordance with the present disclosure is illustrated and generally identified by reference numeral 600. Spinal implant 600 is substantially similar to spinal implant 400, and therefore, only the differences therebetween will be described in detail in the interest of brevity. Body 602 includes a plurality of enlarged orifices 626c defined through opposed side surfaces 616, 618. In this manner, the plurality of enlarged orifices 626c is interposed between each orifice 626b defined through opposed side surfaces 616, 618 such that the orifices of the plurality of enlarged orifices 626c and orifices 626b are arranged in an alternating pattern. Although illustrated as having a generally diamond shaped cross-section, it is contemplated that the plurality of enlarged orifices 626c may include any suitable cross-section, such as circular, oval, square, hexagonal, rectangular, or the like.

As can be appreciated, the features of spinal implants 500 and 600 may be combined, such that spinal implant 500 may further include the plurality of enlarged orifices 626c defined through opposed side surfaces 516, 518, or spinal implant 600 may include the first and second pluralities of enlarged orifices 526c, 526d defined through top and bottom surfaces 612, 614.

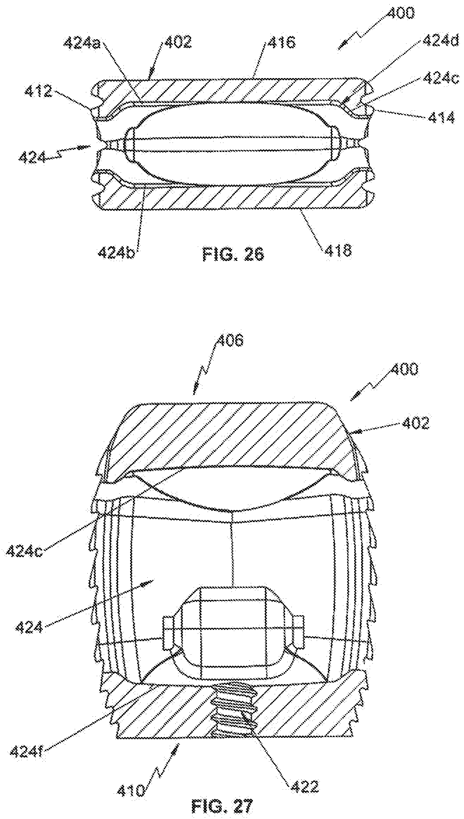

With reference to FIGS. 26 and 27, front and bottom cross-sectional views of spinal implant 400 are illustrated. The interior dimensions of through-bore 424 increase in a direction towards opposed side walls 416, 418. In this manner, through-bore 424 is configured to receive a greater amount of biological material than is possible with a through-bore having planar side walls. Through-bore 424 includes a pair of opposed interior surfaces 424a and 424b adjacent opposed side surfaces 416, 418. Although generally illustrated as defining a planar configuration, it is contemplated that opposed interior surfaces 424a, 424b may include any suitable configuration, such as convex, concave, may approximate each other in a cephalad or caudal direction, or approximate each other in a distal or proximal direction, or any combination thereof. As best illustrated in FIG. 26, through-bore 424 includes a bevel or undercut 424c extending in an interior direction from each of opposed side surfaces 416, 418 and towards a respective opposed interior surface 424a, 424b. The undercut 424c aids in retaining the bone growth material therein, reducing the possibility that the bone growth material may become separated or dislodged from spinal implant 400. Further still, providing spinal implant 400 with an undercut 424c allows implant 400 to house a larger volume of bone growth material or other biologics as compare to a spinal implant lacking an undercut. Although illustrated as including a fillet 424d joining undercut 424c and opposed interior surfaces 424a, 424b, it is contemplated that the intersection of undercut 424c and a respective opposed interior surface 424a, 424b may include any suitable joining feature, such as a sharp corner, bevel, or the like.

As best illustrated in FIG. 27, through-bore 424 includes generally planar end surfaces 424e and 424f at leading and trailing ends 406, 410, respectively. As can be appreciated, each of planar end surfaces 424e, 424f may include any suitable profile, such as concave, convex, may approximate one another in a cephalad direction, may approximate one another in a caudal direction, may approximate one another in a distal direction, a proximal direction, or any combination thereof.

As can be appreciated, manufacturing spinal implants 10, 110, 210, 310, 400, 500, and 600 using standard machining methods (e.g., lathe, mill, electrical discharge machining, etc.) would be difficult. In view of this, it is contemplated that spinal implants 10, 110, 210, 310, 400, 500, and 600 may be manufactured by means of additive manufacturing methods (e.g., shape deposition manufacturing, selective laser powder processing, direct metal laser sintering, selective laser sintering, selective laser melting, selective heat sintering, or electron-beam melting. Other manufacturing methods include VAT photopolymerisation and material jetting that are methods that do not form part of the invention but represent background art that is useful for understanding the invention. As each of spinal implants 10, 110, 210, 310, 400, 500, and 600 may be constructed in a similar fashion, only the method of constructing spinal implant 400 utilizing additive manufacturing methods will be described herein in the interest of brevity. In one non-limiting embodiment, spinal implant 400 may be manufactured using Selective Laser Powder Processing (SLPP). SLPP utilizes powdered metal and a laser which sinters or cures the metal in a selective fashion according to the design intent in thin layers. In embodiments, the layers may have a thickness of about 250 µm. Spinal implant 400 is built layer by layer to allow for more design options and features which would be difficult to be machined using conventional methods. Specifically, a first layer of powder is applied to a specialized build plate, at which point the laser cures portions of the powder according to the design intent. At this point, a second layer is applied to the build plate and the laser is again used to cure selective portions of this second layer. This process is repeated until spinal implant 400 is fully formed. Once spinal implant 400 is fully formed, uncured powder is removed using compressed air or other similar means. Next, post machining is performed on spinal implant 400 to remove any burrs or similar imperfections embedded within spinal implant 400 during the additive manufacturing process. In embodiments, the burrs are removed by means of buffer wheels, clippers, files, or the like. Once de-burred, spinal implant 400 is heat treated, and thereafter, media blasted using aluminum oxide. Thereafter, spinal implant 400 is immersed in a hydrofluoric bath to strip the aluminum oxide therefrom. Finally, spinal implant 400 is inspected by quality control personnel (or using automated means), cleaned via ultrasonic cleaning, dried, and packaged. Additionally, using SLPP, it is contemplated that spinal implant 400 may be customized for a designated patient. For a detailed description of exemplary manufacturing methods, reference can be made to U.S. Patent No. 8,590,157, issued on November 6, 2013 to Kruth et al.

Each of spinal implants 10, 110, 210, 310, 400, 500, and 600 may be constructed from titanium, a titanium-alloy, a cobalt-chromium alloy, a ceramic, Polyetheretherketone, or any other suitable biocompatible material. It is also contemplated that spinal implants 10, 110, 210, 310, 400, 500, and 600 may be manufactured using a three-dimensional printer utilizing a biocompatible polymer.

It is envisioned that the manufacturing processes and orifice designs detailed above may be utilized to form various other medical devices known in the art. In this manner, the additive manufacturing process detailed above may be employed to form corpectomy devices, fixed spinal implants, expandable spinal implants, bone screws, cervical implants, and the like. Similarly, the orifice designs detailed above may be formed in any of the beforementioned medical devices that would benefit from an increased ability to fuse with bone. Examples of such devices may be found in the following commonly owned references: U.S. Patent No. 8,585,761 to Theofilos, U.S. Patent No. 8,673,011 to Theofilos et al., U.S. Application Serial No. 14/936,911 to Sutterlin et al., U.S. Patent No. 8,801,791 to Soo et al., U.S. Patent No. 8,439,977 to Kostuik et al., U.S. Patent Application Publication No. 2010/0100131 to Wallenstein, U.S. Patent Application Publication No. 2012/0179261 to Soo, U.S. Patent No. 8,449,585 to Wallenstein et al., U.S. Patent No. 8,814,919 to Barrus et al., U.S. Patent No. 5,733,286 to Errico et al., and U.S. Patent Application Publication No. 2013/0046345 to Jones et al.

It is contemplated that any of the disclosed embodiments of the spinal implant may be formed from a molybdenum rhenium alloy or other similar alloy. As can be appreciated, a spinal implant formed from molybdenum rhenium alloy may be constructed using conventional techniques or the additive manufacturing technique described hereinabove using molybdenum and rhenium in powder form. In embodiments, the molybdenum rhenium alloy may include between 40 to 51% of molybdenum and rhenium, although other suitable percentages may be utilized depending upon the needs of the additive manufacturing process being employed. For example, it is contemplated that the molybdenum rhenium alloy may include approximately 52% to 70% molybdenum and 30% to 48% rhenium. In one specific example, it is envisioned that the molybdenum rhenium alloy may include approximately 52.5% molybdenum and approximately 47.5% rhenium.

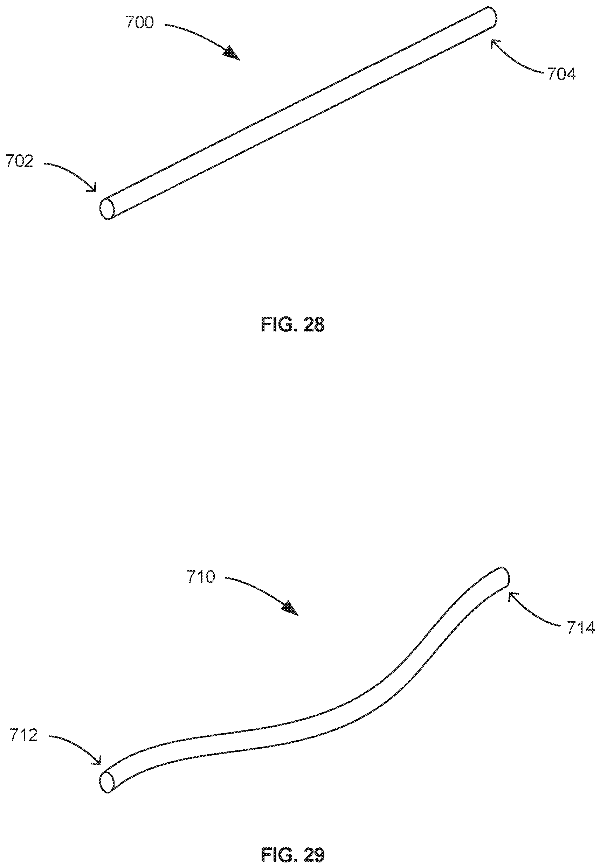

With reference to FIG. 28, a spinal rod provided in accordance with the present disclosure is illustrated and is generally identified by reference numeral 700. The spinal rod 700 extends between a caudal portion 702 and an opposite, cephalad portion 704 and may be formed from any suitable material such as titanium, titanium-alloy, a cobalt-chromium alloy, a ceramic, polyetheretherketone, etc. In one non-limiting embodiment, the spinal rod 700 is formed from a molybdenum rhenium alloy or other similar alloy, and in other embodiments is formed from a molybdenum rhenium alloy containing between 40 to 51% of molybdenum and rhenium. In other examples, it is contemplated that the molybdenum rhenium alloy may include approximately 52% to 70% molybdenum and 30% to 48% rhenium. In one specific example, it is envisioned that the molybdenum rhenium alloy may include approximately 52.5% molybdenum and approximately 47.5% rhenium.

As can be appreciated, the spinal rod 700 may be formed using any of the additive manufacturing techniques described hereinabove using molybdenum and rhenium in powder form. In embodiments where the spinal rod 700 is formed using additive manufacturing, the percentage of molybdenum and rhenium in the molybdenum rhenium alloy may vary depending upon the needs of the additive manufacturing technique being utilized.

It is also envisioned that the spinal rod may be customized for a particular application with a specific configuration as illustrated in FIG. 29. The spinal rod 710 extends between a caudal portion 712 and an opposite, cephalad portion 714 and may be formed having the required shape without the need for bending or other post machining processes to conform to the patient's body. Thus, before manufacturing the spinal rod 710, the desired geometric shape (e.g., the length, number of bends, radii of bends, etc.) is identified by the clinician. At this point, the desired material is chosen by the clinician (e.g., titanium, a molybdenum rhenium alloy, a cobalt chrome alloy, etc.) Using specific geometric information and the selected material, the spinal rod 710 may be custom formed using an additive manufacturing process, thereby eliminating or limiting manipulation or machining of the spinal rod 710 during or after manufacturing.

It is contemplated that the clinician may utilize a software suite capable of determining the ideal geometric shape of the spinal rod 710, such as Surgimap^{®}, marketed and sold by Nemaris Inc. ^{™}. In this manner, images of a patient are uploaded to the software suite using any suitable means, such as from the Electronic Medical Records (EMR) database via the internet, the intranet, etc., or by a computer readable medium such as a memory stick, compact disc, etc. As can be appreciated, any suitable imaging modality may be utilized to obtain the patient images, such as X-Ray, Magnetic Resonance Imaging, etc. Using the software suite, the clinician identifies desired anatomical landmarks and a representative spinal rod 710 is overlaid on the image. Once the representative spinal rod 710 is created, the clinician may select the material from which the spinal rod 710 may be formed, select the diameter of the spinal rod 710, and adjust the bend factor according to any desired level. At this point, the clinician can order a template corresponding to the spinal rod 710 designed using the software suite, such that a custom spinal rod 710 may be formed according to the template. It is contemplated that the software suite may be utilized to generate a spinal rod profile from which the spinal rod 710 may be formed using any of the additive manufacturing techniques disclosed hereinabove.

In some embodiments, a rod may be formed with a varying degree of stiffness. For instance, a rod formed with one material may be modified to include an extension formed with a second material utilizing an additive manufacturing technique. In one example, a molybdenum rhenium alloy rod may be modified to include a titanium extension 3-D printed onto the existing rod. In this manner, a single rod is produced with a stiffness that varies between the MoRe alloy part and the titanium part.

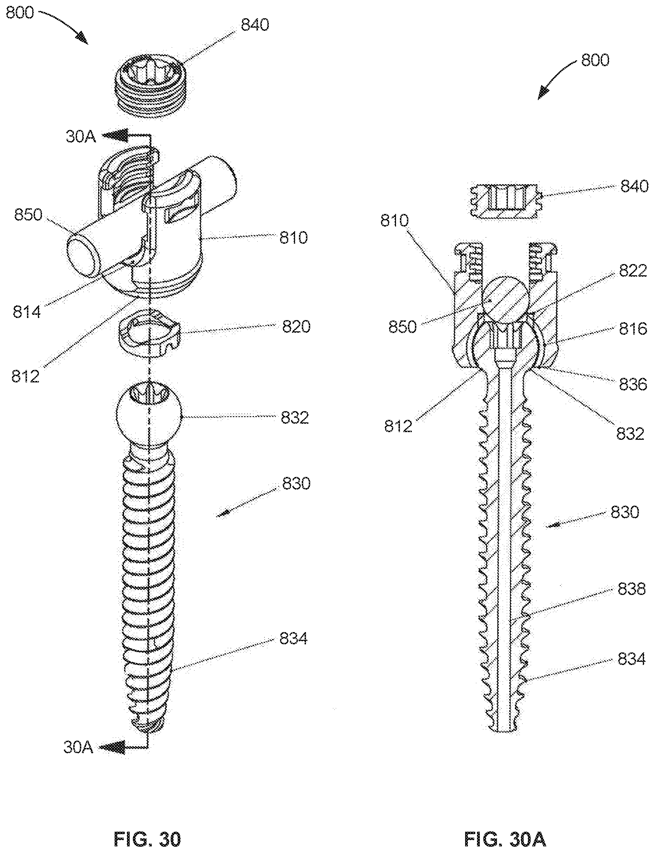

Turning now to FIGS. 30 and 30A, a bone screw assembly provided in accordance with the present disclosure is illustrated and generally identified by reference numeral 800. Although generally illustrated as being a polyaxial pedicle screw, it is contemplated that the bone screw assembly 800 may be any suitable bone screw capable of engaging bone. The bone screw assembly 800 includes a housing 810, an anvil 820, and a bone screw member 830. The bone screw member 830 includes a head 832 and a threaded shaft 834 extending therefrom. The housing 810 defines an aperture 812 therein that includes a shape that is complementary to both the anvil 820 and the head 832 of the bone screw member 830. In this manner, the aperture 812 is configured to enable pivoting and rotation of the head 832 of the bone screw member 830 while the head 832 is positioned therein. The head 832 defines an outer diameter that is larger than a diameter of the aperture 812 and the threaded shaft 834 defines an outer diameter that is smaller than the diameter of the aperture 812 thereby inhibiting the head 832 from passing therethrough while enabling the threaded shaft 834 to pass therethrough. A proximal end portion of the housing 810 includes a U-shaped channel 814 defined therein that is configured to receive a set screw 840 and a spinal rod 850. The U-shaped channel 814 defines a threaded portion that is configured to threadably engage the set screw 840 and the head 832 defines an outer diameter that is larger than the opening of the U-shaped channel 814, such that the head 832 is inhibited from passing through the U-shaped channel 814.

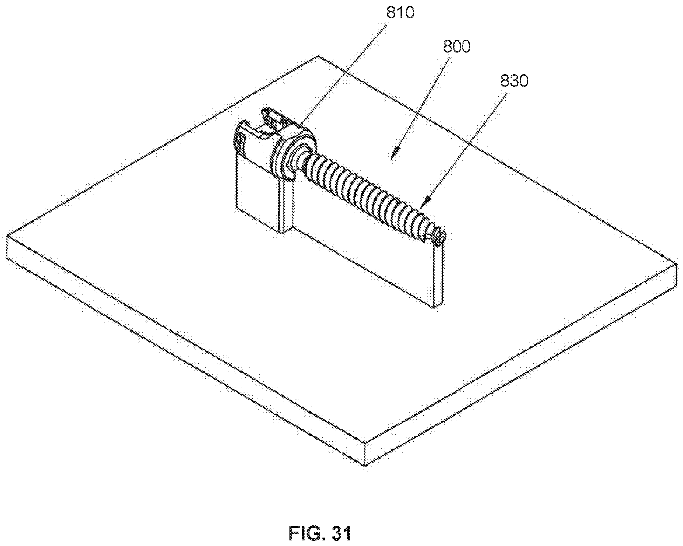

With additional reference to FIG. 31, it is contemplated that the bone screw assembly 800 may be formed using any of the above-described additive manufacturing processes. In this manner, the bone screw assembly 800 may be monolithically formed such that each of the components of the bone screw assembly 800 (e.g., housing 810, anvil 820, bone screw member 830) may be formed simultaneously (e.g., monolithically) such that when the additive manufacturing process is complete, the bone screw assembly is in a fully assembled state. Thus, no additional steps are required to assembly the bone screw assembly 800. As can be appreciated, not only does this manufacturing process reduce manufacturing steps, but also permits the manufacture of designs that could not be assembled using traditional machining and assembly methods.

Therefore, as manufactured in accordance with any of the additive manufacturing processes disclosed hereinabove, a feature of the first unitary, monolithic part is configured and dimensioned to nest and be housing within a cavity of the second unitary, monolithic part such that the two parts are movable relative to one another but are not separable from one another. As can be appreciated, this approach eliminates the need for features to mechanically assemble parts and then retain the parts in an assembled condition. It is contemplated that the anvil 820 may also be made during the manufacturing process to be positioned within the housing 810 adjacent to the head 832 of the bone screw member 830. The set screw 840 is positionable within the housing 810 and is threadably engageable therewith. Each of the housing 810, anvil 820, and head 832 of the bone screw member 830 defines a cleaning slot 816, 822, and 836, respectively, that enable support material to escape during post procedure steps (e.g., the support material may escape during a cleaning procedure). In embodiments, the bone screw assembly 800 may be fully assembled when the anvil 820 and the head 832 of the bone screw member 830 is positioned within the housing 810.

Using any of the additive manufacturing processes disclosed hereinabove, it is contemplated that a construct of spinal rods and bone screw assemblies may be formed simultaneously (e.g., a plurality of bone screws attached to a spinal rod may be 3-D printed) such that the construct may be secured to a patient's spinal column as a whole and the spinal rod secured with set screws at each bone screw to finalize the placement of the construct. In this manner, additive manufacturing may be utilized to quickly and accurately manufacture a spinal rod system or construct, rather than assembling multiple components to complete the construct.

The bone screw assembly 800 may be formed from any suitable material such as titanium, titanium-alloy, a cobalt-chromium alloy, a ceramic, polyetheretherketone, etc. In one non-limiting embodiment, the bone screw assembly 800 is formed from a molybdenum rhenium alloy or other similar alloy, and in embodiments is formed from a molybdenum rhenium alloy containing between 40 to 51% of molybdenum and rhenium. In some examples, it is contemplated that the molybdenum rhenium alloy may include approximately 52% to 70% molybdenum and 30% to 48% rhenium. In one specific example, it is envisioned that the molybdenum rhenium alloy may include approximately 52.5% molybdenum and approximately 47.5% rhenium.

For a detailed description of bone screw assemblies manufactured using additive manufacturing techniques, reference may be made to co-pending U.S. Patent Application Serial No. 15/643,603, titled "Surgical Implant and Methods of Additive Manufacturing," filed on July 7, 2017.

It is envisioned that the methods and materials described herein may be utilized in the construction of adjustable spinal implants (e.g., corpectomy cages), such as those described in U.S. Patent No. 9,707,096 to Sutterlin, III et al. and U.S. Patent Application Publication No. 2016/0058575 to Sutterlin, III et al., filed on November 10, 2015, and expandable spinal implants, such as those described in U.S. Patent Application Serial No. 15/657,796 to Ludwig et al., filed on July 24, 2017.

In embodiments, the bone screws and bone screw assemblies described herein may include a combination of cancellous and cortical threads, amongst others.

It is contemplated that the methods and materials described herein may be utilized to construct cervical plates, such as those described in U.S. Patent Application Publication No. 2016/0213405 to Moore et al, filed on January 27, 2016. In embodiments, the cervical plates may include an I-beam shape, a T-shape, amongst others. Further, it is envisioned that the cervical plate manufactured in accordance with the methods and using the materials described herein may include a thinner cross-sectional thickness than is ordinarily possible using known techniques and material.

In embodiments, the methods and materials described herein may be utilized to construct tapered rods, such as those described in U.S. Patent No. 9,795,413 to Barrus. It is contemplated that the rods may include an oval shape that transitions to a round shape. In this manner, the stiffness of the rod may be adjusted depending upon the cross-sectional profile of the rod along its length. In embodiments, the diameter of the rod may transition from 6mm to 4mm (e.g., from a diameter of a lumbar rod to a diameter of a cervical rod) at various locations to enable the rod to be utilized in multiple applications.

It is envisioned that the devices described herein may include myriad synthetic or naturally occurring pharmaceutical or biological agents in liquid or gel formations depending upon the particular application. Drugs may be administered for any actual or potential therapeutic, prophylactic or other medicinal purpose. Such drugs may include, e.g., analgesics, anesthetics, antimicrobial agents, antibodies, anticoagulants, antifibrinolytic agents, anti-inflammatory agents, antiparasitic agents, antiviral agents, cytokines, cytotoxins or cell proliferation inhibiting agents, chemotherapeutic agents, radiolabeled compounds or biologics, hormones, interferons, and combinations thereof.

Therapeutic agents may include chemotherapeutic agents (for example, paclitaxel, vincristine, ifosfamide, dacttinomycin, doxorubicin, cyclophosphamide, and the like), bisphosphonates (for example, alendronate, pamidronate, clodronate, zoledronic acid, and ibandronic acid), analgesics (such as opioids and NSAIDS), anesthetics (for example, ketoamine, bupivacaine and ropivacaine), tramadol, and dexamethasone. In embodiments, the devices described herein may include an agent useful in radiotherapy in, e.g., beads.

In other embodiments, the devices described herein may include radiotherapy agents such as radiolabeled antibodies, radiolabeled peptide receptor ligands, or any other radiolabeled compound capable of specifically binding to the specific targeted cancer cells.

In addition, the devices described herein may include drugs used in the management of pain and swelling that occurs following the implantation surgery. For example, the devices described herein may release an effective amount of an analgesic agent alone or in combination with an anesthetic agent. As yet another alternative, the devices described herein may be used to deliver drugs which help minimize the risk of infection following implantation. For example, the devices described herein may release one or more antibiotics (for example, cefazolin, cephalosporin, tobramycin, gentamycin, etc.) and/or another agent effective in preventing or mitigating biofilms (for example, a quorum-sensing blocker or other agent targeting biofilm integrity). Bacteria may form biofilms on the surface of the above described devices, and these biofilms may be relatively impermeable to antibiotics. Accordingly, systemically administered antibiotics may not achieve optimal dosing where it is most needed. However, it is contemplated that the devices described herein may enable the delivery of the desired dose of antibiotic precisely when and where needed. In certain circumstances, the antibiotic may be delivered beneath the biofilm.

The scope of the present invention as defined by the appended claims.

## Claims

1. A method of manufacturing a spinal rod, the method comprising the following steps:
using a software for identifying a required geometric shape of the spinal rod by obtaining a digital image of a patient and overlaying a representative spinal rod on a plurality of anatomical landmarks on the digital image; and
forming at least part of the spinal rod using a directed energy deposition process, a powder bed fusion process, a binder jetting process or a shape deposition manufacturing process, the process comprising:
selecting a molybdenum rhenium alloy consisting of 52% to 70% molybdenum and 30% to 48% rhenium, the molybdenum rhenium alloy being used to form the at least part of the spinal rod; and
curing a plurality of layers of the selected molybdenum rhenium alloy to form the at least part of the spinal rod having the required geometric shape without the need for bending or other post machining processes to conform to a patient's body.

2. The method according to claim 1, wherein forming the at least part of the spinal rod includes curing the plurality of layers so that the required geometric shape includes at least one bend.

3. The method according to claim 2, wherein forming the at least part of the spinal rod includes forming the at least one bend of the spinal rod with a predetermined radius.

4. The method according to any one of claims 1-3, wherein the at least part of the spinal rod is a second part of the spinal rod, the method further comprising identifying a first stiffness for a first part of the spinal rod formed with a first material and a second stiffness for a second part of the spinal rod formed with the molybdenum rhenium alloy, the first stiffness being different from the second stiffness, the spinal rod being formed to include the first stiffness in the first part and the second stiffness in the second part.

5. The method according to claim 4, wherein identifying the required geometric shape of the spinal rod includes assigning the first part of the spinal rod a first diameter and the second part of the spinal rod a second diameter different from the first diameter, the identified shape being arranged such that the spinal rod tapers from the first part to the second part.

6. The method according to claim 4, further comprising selecting titanium for the first part of the spinal rod.

7. The method according to any one of claims 1-3, wherein the forming of the at least part of the spinal rod includes modifying the rod to include an extension formed with a second material.

8. The method according to any one of claims 1-7, further comprising forming a portion of the spinal rod using a process other than additive manufacturing.

9. The method according to any one of claims 1-7, further comprising forming a portion of the spinal rod separate from the at least part of the spinal rod, the portion formed through the selection of a second material different from the molybdenum rhenium alloy.

## Patentansprüche

1. Verfahren zur Herstellung eines Wirbelsäulenstabs, wobei das Verfahren die folgenden Schritte umfasst:
Verwenden einer Software zum Identifizieren der erforderlichen geometrischen Form des Wirbelsäulenstabs, indem ein digitales Bild eines Patienten erstellt und ein repräsentativer Wirbelsäulenstab auf einer Vielzahl von anatomischen Orientierungspunkten im digitalen Bild überlagert wird; und
Bilden mindestens eines Teils des Wirbelsäulenstabs unter Verwendung eines gerichteten Energieabscheidungsverfahrens, eines Pulverbettfusionsverfahrens, eines Bindemittelstrahlverfahrens oder eines Formabscheidungsherstellungsverfahrens, wobei das Verfahren Folgendes umfasst:
Auswählen einer Molybdän-Rhenium-Legierung, die aus 52 % bis 70 % Molybdän und 30 % bis 48 % Rhenium besteht, wobei die Molybdän-Rhenium-Legierung verwendet wird, um den mindestens einen Teil des Wirbelsäulenstabs zu bilden; und
Aushärten einer Vielzahl von Schichten der ausgewählten Molybdän-Rhenium-Legierung, um den mindestens einen Teil des Wirbelsäulenstabs mit der erforderlichen geometrischen Form zu bilden, ohne dass Biegen oder andere Nachbearbeitungsprozesse notwendig sind, um sich dem Körper eines Patienten anzupassen.

2. Verfahren nach Anspruch 1, wobei das Bilden des mindestens Teils des Wirbelsäulenstabs das Aushärten der Vielzahl von Schichten umfasst, so dass die erforderliche geometrische Form mindestens eine Biegung enthält.

3. Verfahren nach Anspruch 2, wobei das Bilden des mindestens einen Teils des Wirbelsäulenstabs das Bilden der mindestens einen Biegung des Wirbelsäulenstabs mit einem vorbestimmten Radius enthält.

4. Verfahren nach einem der Ansprüche 1 bis 3, wobei der mindestens eine Teil des Wirbelsäulenstabs ein zweiter Teil des Wirbelsäulenstabs ist, wobei das Verfahren ferner das Identifizieren einer ersten Steifigkeit für einen ersten Teil des aus einem ersten Material ausgebildeten Wirbelsäulenstabs und einer zweiten Steifigkeit für einen zweiten Teil des aus der Molybdän-Rhenium-Legierung ausgebildeten Wirbelsäulenstabs umfasst, wobei die erste Steifigkeit sich von der zweiten Steifigkeit unterscheidet, wobei der Wirbelsäulenstab so ausgebildet ist, dass die erste Steifigkeit im ersten Teil und die zweite Steifigkeit im zweiten Teil enthalten ist.

5. Verfahren nach Anspruch 4, wobei das Identifizieren der erforderlichen geometrischen Form des Wirbelsäulenstabs das Zuweisen eines ersten Durchmessers zum ersten Teil des Wirbelsäulenstabs und eines zweiten Durchmessers, der sich vom ersten Durchmesser unterscheidet, zum zweiten Teil des Wirbelsäulenstabs enthält, wobei die identifizierte Form so angeordnet ist, dass sich der Wirbelsäulenstab vom ersten Teil zum zweiten Teil verjüngt.

6. Verfahren nach Anspruch 4, das ferner das Auswählen von Titan für den ersten Teil des Wirbelsäulenstabs umfasst.

7. Verfahren nach einem der Ansprüche 1 bis 3, wobei das Bilden des mindestens einen Teils des Wirbelsäulenstabs das Modifizieren des Stabs enthält, um eine aus einem zweiten Material ausgebildete Verlängerung aufzunehmen.

8. Verfahren nach einem der Ansprüche 1 bis 7, das ferner das Bilden eines Abschnitts des Wirbelsäulenstabs unter Verwendung eines anderen Verfahrens als der additiven Fertigung umfasst.

9. Verfahren nach einem der Ansprüche 1 bis 7, das ferner das Bilden eines Abschnitts des Wirbelsäulenstabs umfasst, der von dem mindestens einen Teil des Wirbelsäulenstabs getrennt ist, wobei der Abschnitt durch die Auswahl eines zweiten Materials ausgebildet ist, das sich von der Molybdän-Rhenium-Legierung unterscheidet.

## Revendications

1. Procédé de fabrication d'une tige vertébrale, le procédé comprenant les étapes suivantes :
utiliser un logiciel pour identifier une forme géométrique requise de la tige vertébrale en obtenant une image numérique d'un patient et en superposant une tige vertébrale représentative sur une pluralité de repères anatomiques sur l'image numérique ; et
former au moins une partie de la tige vertébrale à l'aide d'un procédé de dépôt d'énergie dirigée, d'un procédé de fusion sur lit de poudre, d'un procédé d'impression à jet de liant ou d'un procédé de fabrication par dépôt de forme, le procédé comprenant :
sélectionner un alliage de molybdène-rhénium consistant en de 52 % à 70 % de molybdène et de 30 % à 48 % de rhénium, l'alliage de molybdène-rhénium étant utilisé pour former au moins une partie de la tige vertébrale ; et
durcir une pluralité de couches de l'alliage molybdène-rhénium sélectionné pour former au moins une partie de la tige vertébrale ayant la forme géométrique requise sans avoir besoin de procéder à un cintrage ou à d'autres processus d'usinage ultérieurs pour l'adapter au corps d'un patient.

2. Procédé selon la revendication 1, dans lequel la formation d'au moins une partie de la tige vertébrale comprend le durcissement de la pluralité de couches de telle sorte que la forme géométrique requise comprenne au moins un coude.

3. Procédé selon la revendication 2, dans lequel la formation d'au moins une partie de la tige vertébrale comprend la formation d'au moins un coude de la tige vertébrale avec un rayon prédéterminé.

4. Procédé selon l'une quelconque des revendications 1 à 3, dans lequel la partie au moins de la tige vertébrale est une deuxième partie de la tige vertébrale, le procédé comprenant en outre l'identification d'une première rigidité pour une première partie de la tige vertébrale formée avec un premier matériau et d'une deuxième rigidité pour une deuxième partie de la tige vertébrale formée avec l'alliage de molybdène et de rhénium, la première rigidité étant différente de la deuxième rigidité, la tige vertébrale étant formée de manière à inclure la première rigidité dans la première partie et la deuxième rigidité dans la deuxième partie.

5. Procédé selon la revendication 4, dans lequel l'identification de la forme géométrique requise de la tige vertébrale comprend l'attribution à la première partie de la tige vertébrale d'un premier diamètre et à la deuxième partie de la tige vertébrale d'un deuxième diamètre différent du premier diamètre, la forme identifiée étant agencée de telle sorte que la tige vertébrale s'effile de la première partie vers la deuxième partie.

6. Procédé selon la revendication 4, comprenant en outre la sélection de titane pour la première partie de la tige vertébrale.

7. Procédé selon l'une quelconque des revendications 1 à 3, dans lequel la formation d'au moins une partie de la tige vertébrale comprend la modification de la tige pour inclure une extension formée avec un deuxième matériau.

8. Procédé selon l'une quelconque des revendications 1 à 7, comprenant en outre la formation d'une partie de la tige vertébrale à l'aide d'un procédé autre que la fabrication additive.

9. Procédé selon l'une quelconque des revendications 1 à 7, comprenant en outre la formation d'une partie de la tige vertébrale séparée de la partie au moins de la tige vertébrale, la partie étant formée par la sélection d'un deuxième matériau différent de l'alliage molybdène-rhénium.
